# EUROPEAN PATENT SPECIFICATION

(11) **EP 0 794 764 B1**
(45) Date of publication and mention of the grant of the patent: **31.03.1999**
(21) Application number: 95940840.2
(22) Date of filing: 21.11.1995
(51) Int. Cl.: A61K 7/48

(54) **TOPICAL SKIN CARE COMPOSITIONS CONTAINING THICKENED POLYOL CARBOXYLIC ACID ESTERS AS SKIN CONDITIONING AGENTS**
TOPISCHE HAUTPFLEGEMITTEL ENTHALTEND VERDICKTE POLYOLESTERN VON CARBONSAÜREN ALS HAUT KONDITIONIERENDE AGENTIEN
COMPOSITIONS DE SOIN POUR LA PEAU A USAGE LOCAL, DONT LES AGENTS REVITALISANTS SONT DES ESTERS D'ACIDE CARBOXYLIQUE POLYOLIQUE EPAISSIS

(30) Priority: 28.11.1994 US 345154; 15.11.1995 US 538832
(43) Date of publication of application: 17.09.1997
(73) Proprietor: THE PROCTER & GAMBLE COMPANY, Cincinnati, Ohio 45202 (US)
(72) Inventor: DOUGHTY, Darrell, Gene, Cincinnati, OH 45249 (US); GATTO, Joseph, Anthony, Cincinnati, OH 45249 (US); WEISGERBER, David, John, Cincinnati, OH 45239 (US); SCHWARTZ, James, Robert, West Chester, OH 45069 (US)
(74) Representative: Woof, Victoria
(86) International application number: US9515374
(87) International publication number: WO9616636

(56) References cited:
- EP-A- 0 466 410
- EP-A- 0 646 368
- WO-A-91/15963
- WO-A-93/08840

## Description

The present invention relates to skin care compositions containing a skin conditioning agent comprising a nonocclusive, liquid polyol carboxylic acid ester having a complete melting point of less than 30°C and a solid polyol carboxylic acid ester having a complete melting point greater than 30°C, and a topical carrier for the skin conditioning agent.

### BACKGROUND OF THE INVENTION

The treatment of human skin with various agents has been undertaken for many years with the goal being to keep the skin in a smooth and supple condition. Skin has the tendency to dry out when exposed to low humidity or to harsh detergent solutions for extended periods of time. From a physiological standpoint, dryness is a measure of the water content of the skin. Under normal conditions, the water content and vapor pressure of the epidermis are higher than those of the surrounding air, with consequent evaporation of water from the skin surface. Skin becomes dry because of excessive loss of water from its surface which results in loss of water from the stratum corneum. Low humidity speeds up this process, exacerbating the drying of skin.

Continuous and prolonged immersion in soap or detergent solutions can contribute to dryness of the stratum corneum. The reason for this is that the surfactant medium promotes dissolution of the skin surface and horny layer lipids, and the dissolution of the hygroscopic water-soluble components in the skin.

In attempts to alleviate or prevent the aforementioned conditions, many different emollient materials have been suggested for topical application to the skin. See, for example, Sagarin, *Cosmetics, Science and Technology*, 2nd Edition, vol. 1, pages 34-36 (1972). Skin conditioning agents are believed to increase the state of hydration of the skin by altering the rate of diffusion of water from the lower epidermal and dermal layers, the rate of evaporation of water from the skin's surface, and the ability of the corneum layer to hold moisture.

Various materials are purported to be effective skin conditioners. See *CTFA Cosmetic Ingredient Handbook*, Second Edition, 1992. However, the most effective and widely used materials, such as glycerol, suffer from negative aesthetic qualities, such as greasiness or stickiness Conversely, materials with better aesthetics tend to be ineffective as skin conditioners. Additionally, European Patent No. 458,600 B1, published March 2, 1994, discloses occlusive skin care compositions containing a polyol fatty acid polyester having at least 4 free hydroxyl groups, at least 60% of which are esterified with one or more fatty acids having from 8 to 22 carbon atoms. However, these compositions have the disadvantage of being heavy and occlusive, thereby clogging the skin's pores and preventing the flow of oxygen. Therefore, the need exists for materials which can meet both efficacy and aesthetic criteria without being heavy and occlusive. Such materials would find immediate application for example, in a wide variety of skin care compositions.

It has been found in the present invention that skin care compositions containing certain nonocclusive, liquid polyol carboxylic acid esters as the skin conditioning agent provide a skin conditioning benefit without the aesthetic negatives and undesirable occlusive effects mentioned herein. It has also been found that when these liquid esters are used in combination with a solid polyol carboxylic acid ester thickening agent, as defined herein, that skin conditioning benefits without aesthetic negatives and undesirable occlusive effects are also obtained. These thickened liquids have been found to be especially substantive to skin.

It is an object of the present invention to provide skin conditioning agents comprising nonocclusive liquid polyol carboxylic acid esters which possess both excellent skin conditioning and aesthetic properties.

It is another object of the present invention to provide skin care compositions containing these nonocclusive skin conditioning agents comprising liquid polyol carboxylic acid esters, such that these compositions possess both excellent skin conditioning and aesthetic properties.

It is another object of the present invention to provide skin conditioning agents which possess both excellent skin conditioning and aesthetic properties, comprising nonocclusive liquid polyol carboxylic acid esters and solid polyol carboxylic acid ester thickening agents for the esters.

It is another object of the present invention to provide skin care compositions containing these conditioning agents comprising nonocclusive liquid polyol carboxylic acid esters and solid polyol carboxylic acid ester thickening agents for the esters, such that these compositions possess both excellent skin conditioning and aesthetic properties.

These and other objects will become readily apparent from the detailed description which follows.

### SUMMARY OF THE INVENTION

The present invention relates to a topical skin care composition comprising:
(A) from 0.1% to 99.9% of a skin conditioning agent comprising:
   (i) from 50% to 99.99%. based on the total weight of said skin conditioning agent, of a nonocclusive, liquid polyol carboxylic acid ester having a polyol moiety and at least 2 carboxylic acid moieties, wherein the polyol moiety is selected from sugars and sugar alcohols containing from 4 to 11 hydroxyl groups, and wherein each carboxylic acid moiety has from 8 to 22 carbon atoms, and wherein said ester has a complete melting point of less than 30°C; and
   (ii) from 0.01% to 50%, based on the total weight of said skin conditioning agent of a solid polyol carboxylic acid ester having a polyol moiety and at least 2 carboxylic acid moieties wherein the polyol moiety contains at least 4 hydroxyl groups, wherein the carboxylic acid moiety consists essentially of (a) C12 or higher unsaturated carboxylic acid moieties or a mixture of said unsaturated moieties and C2 to C12 saturated carboxylic acid moieties, and (b) C20 or higher saturated carboxylic acid moieties, the molar ratio of (a) to (b) being from 3:5 to 1:7, wherein at least 4 of the hydroxyl groups of the polyol moiety are esterified; and wherein the complete melting point of the solid polyol carboxylic acid ester is above 30°C; and
(B) from 0.1% to 99.9% of a topical carrier for said skin conditioning agent.

All percentages and ratios used herein are by weight and all measurements made are at 25°C, unless otherwise designated. The invention hereof can comprise, consist of, or consist essentially of the essential as well as optional ingredients and components described herein.

### DETAILED DESCRIPTION OF THE INVENTION

The term "topical skin care composition" as used herein means a composition suitable for application to the human skin surface. The term is used to encompass a wide variety of personal care, beauty care, and cosmetic compositions. Nonlimiting examples of topical skin care compositions include skin conditioning lotions and creams, skin protectant compositions, hand and body lotions, sunscreen compositions, anti-acne compositions, skin renewal products, make-ups, foundations, toners, lipsticks, lip protectants, cleansers and the like.

The term "nonocclusive" as used herein, means that the material so described does not obstruct the skin surface or block the passage or circulation of air and moisture.

The term "skin conditioning agent", as used herein means a material which provides a "skin conditioning benefit". As used herein, the term "skin conditioning benefit" means to provide a therapeutic or cosmetic benefit to the skin including, but not limited to moisturization, humectancy which is the ability to retain or hold water or moisture in the skin, emolliency, visual improvement of the skin surface, soothing of the skin, softening of the skin, healing of minor cuts, abrasions and burns of the skin, and the like. The foregoing terms are all included under skin conditioning, because a skin conditioning agent can provide one or more of these enumerated and other related benefits.

The term "topical carrier", as used herein, is well-known to one of ordinary skill in the art, and means one or more compatible solid or liquid filler diluents or vehicles which are suitable for administration to a human. The term "compatible", as used herein, means that the components of the topical carrier are capable of being comingled with the components of the present invention, and with each other, in a manner such that there is no interaction which would substantially reduce the efficacy or aesthetics of the skin conditioning composition under ordinary use situations. The topical carrier must be a pharmaceutically acceptable carrier. The term "pharmaceutically-acceptable", as used herein, means that the topical carrier must be of sufficiently high purity and be suitable for use in contact with human skin without undue toxicity, incompatibility, instability, allergic response, and the like.

The term "complete melting point", as used herein means a melting point as measured by the well-known technique of Differential Scanning Calorimetry (DSC). The complete melting point is the temperature at the intersection of the baseline, i.e. the specific heat line, with the line tangent to the trailing edge of the endothermic peak. Typically a scanning temperature of 5°C/minute is used in the present invention in measuring the complete melting points. A technique for measuring complete melting points is more fully described in U.S. Patent No. 5,306,514, to Letton et al., issued April 26, 1994.

### SKIN CONDITIONING AGENT

The present invention comprises from 0.1% to 99.9%, preferably from 0.5% to 20%, and more preferably from 1% to about 10% by weight of a skin conditioning agent.

The skin conditioning agent comprises a nonocclusive liquid polyol carboxylic acid ester and a solid polyol carboxylic acid ester.

The nonocclusive liquid polyol esters are derived from a polyol radical or moiety and one or more carboxylic acid radicals or moieties. In other words, these esters contain a moiety derived from a polyol and one or more moieties derived from a carboxylic acid. These carboxylic acid esters can also be described as liquid polyol fatty acid esters, because the terms carboxylic acid and fatty acid are often used interchangeably by those skilled in the art.

The liquid polyol polyesters employed in this invention comprise certain polyols, especially sugars or sugar alcohols, esterified with at least two fatty acid groups. The polyol starting material, however, preferably has at least about four esterifiable hydroxyl groups. Examples of preferred polyols are sugars, including monosaccharides and disaccharides, and sugar alcohols. Examples of monosaccharides containing four hydroxyl groups are xylose and arabinose and the sugar alcohol derived from xylose, which has five hydroxyl groups, i.e., xylitol. The monosaccharide, erythrose, is suitable in the practice of this invention since it contains three hydroxyl groups, as is the sugar alcohol derived from erythrose, i.e., erythritol, which contains four hydroxyl groups. Suitable five hydroxyl group-containing monosaccharides are galactose, fructose, and sorbose. Sugar alcohols containing six hydroxyl groups derived from the hydrolysis products of sucrose, as well as glucose and sorbose, e.g., sorbitol, are also suitable. Examples of disaccharide polyols which can be used include maltose, lactose, and sucrose, all of which contain eight hydroxyl groups.

The polyols used in the nonocclusive liquid polyol esters of the present invention preferably have from 4 to 12, more preferably from 4 to 11, and most preferably from 4 to 8 hydroxyl groups. Preferred polyols for preparing the polyesters for use in the present invention are selected from erythritol, xylitol, sorbitol, glucose, and sucrose. Sucrose is especially preferred.

The preferred polyol starting material having at least four hydroxyl groups must be esterified on at least two of the hydroxyl groups with a fatty acid containing from 8 to 22 carbon atoms, preferably from 8 to 14 carbon atoms. Examples of such fatty acids include caprylic, capric, lauric, myristic, myristoleic, palmitic, palmitoleic, stearic, oleic, ricinoleic, linoleic, linolenic, eleostearic, arachidic, arachidonic, behenic, and erucic acids. The fatty acids can be derived from naturally occurring or synthetic fatty acids; they can be saturated or unsaturated, including positional and geometrical isomers. However, in order to provide liquid polyesters of the type used herein, at least about half of the fatty acid incorporated into the polyester molecule must be unsaturated fatty acids, short chain saturated fatty acids, or mixtures thereof.

The liquid polyol fatty acid polyesters useful in this invention must contain at least two fatty acid ester groups. It is not necessary that all of the hydroxyl groups of the polyol be esterified with fatty acids, but it is preferable that the polyester contain no more than two unesterified hydroxyl groups. Most preferably, substantially all of the hydroxyl groups of the polyol are esterified with fatty acids, i.e., the polyol moiety is substantially completely esterified. The fatty acids esterified to the polyol molecule can be the same or mixed, but as noted above, a substantial amount of the unsaturated acid ester groups, and or short chain saturated acid ester groups, must be present to provide liquidity.

To illustrate the above points, a sucrose di-fatty acid ester would be suitable, but is not preferred because it has more than two unesterified hydroxyl groups. A sucrose hexa-fatty acid ester would be preferred because it has no more than two unesterified hydroxyl groups. Highly preferred compounds in which all the hydroxyl groups are esterified with fatty acids include the liquid sucrose octa-substituted fatty acid esters.

The following are non-limiting examples of specific nonocclusive liquid polyol fatty acid polyesters containing at least two fatty acid ester groups suitable for use in the present invention: glucose dioleate, the glucose diesters of soybean oil fatty acids (unsaturated), the mannose diesters of mixed soybean oil fatty acids, the galactose diesters of oleic acid, the arabinose diesters of linoleic acid, xylose dilinoleate, sorbitol dioleate, sucrose dioleate, glucose trioleate, the glucose triesters of soybean oil fatty acids (unsaturated), the mannose triesters of mixed soybean oil fatty acids, the galactose triesters of oleic acid, the arabinose triesters of linoleic acid, xylose trilinoleate, sorbitol trioleate, sucrose trioleate, glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate. sucrose tetraoleate, sucrose pentaoletate, sucrose hexaoleate, sucrose hepatoleate, sucrose octaoleate, and mixtures thereof. Preferred are liquid polyol esters selected from sucrose pentaoleate, sucrose hexaoleate, sucrose heptaoleate, sucrose octaoleate, and mixtures thereof. More prefered are sucrose hexaoleate, sucrose heptaoleate, sucrose octaoleate, and mixtures thereof.

The preferred liquid polyol polyesters of the present invention have complete melting points below 30°C, preferably below 27.5°C, and more preferably below 25°C. Complete melting points reported herein are measured by Differential Scanning Calorimetry (DSC).

The polyol fatty acid polyesters suitable for use herein can be prepared by a variety of methods well known to those skilled in the art. These methods include: transesterification of the polyol with methyl, ethyl or glycerol fatty acid esters using a variety of catalysts; acylation of the polyol with a fatty acid chloride; acylation of the polyol with a fatty acid anhydride; and acylation of the polyol with a fatty acid, per se. See U.S. Patent No. 2,831,854;, U.S. Patent No. 4,005,196, to Jandacek, issued January 25, 1977; and U.S. Patent No. 4,005,196, to Jandacek, issued January 25, 1977.

The skin conditioning agents of the present invention also comprise a solid polyol carboxylic acid ester, which can be utilized to thicken the liquid polyol carboxylic acid esters described above.

The solid polyol carboxylic acid ester thickening agent can also be described as a solid polyol fatty acid ester thickening agent, because as mentioned above, the terms carboxylic acid and fatty acid can be used interchangeably. The solid polyol carboxylic acid esters are derived from a polyol radical or moiety ad one or more carboxylic acid radicals or moieties. In other words, these esters contain a moiety derived from a polyol and one or more moieties derived from a carboxylic acid.

It is found that certain polyol carboxylic acid polyesters which have a complete melting point above 30°C are highly effective thickening agents for the liquid polyol carboxylic acid esters described above. Accordingly, these solid polyol carboxylic acid esters can be used as "thickening agents" for blending with materials such as liquid polyol carboxylic acid esters in the formulation of skin conditioning agents.

The solid polyol carboxylic acid ester thickening agents of the present invention have complete melting points above 30°C, preferably above 40°C, more preferably above 50°C, and most preferably above 60°C. Complete melting points reported herein are measured by Differential Scanning Calorimetry (DSC). These solid esters have the ability to trap relatively large amounts of liquids within their crystal structure.

The mixtures of the liquid polyol polyesters and solid polyol polyesters of the present invention are typically prepared by simply mixing the two materials together, generally at a temperature above the melting point of the solid polyol esters.

The percentage by weight of the solid polyol carboxylic acid ester in the skin conditioning agent comprises from 0.01% to 50%, more preferably from 1% to 25%, and most preferably from 2% to 20%. Consequently, the percentage by weight of the liquid polyol carboxylic acid ester in the skin conditioning agent comprises from 50% to 99.99%, preferably from 75% to 99% and more preferably from 80% to 98%. An especially useful mixture for the skin conditioning agent is one comprising 82.3% of the liquid polyol, by weight based on the skin conditioning agent, and 17.7% of the solid polyol, by weight based on the skin conditioning agent.

The solid polyol carboxylic acid polyester thickeners are polyol esters or polyesters wherein the carboxylic acid ester groups of the polyester comprise a combination of: (a) long chain, unsaturated carboxylic acid moieties or a mixture of long chain unsaturated carboxylic acid moieties and short chain saturated carboxylic acid moieties, and (b) long chain saturated carboxylic acid moieties, the ratio of (a) to (b) being from 1 to 15 to 2 to 1, and wherein at least 15%, preferably at least 30%, more preferably at least 50%, and most preferably at least 60% by weight of the total carboxylic acid moieties in the solid polyol polyester are C20 or higher saturated carboxylic acid moieties. The long chain unsaturated carboxylic acid moieties are typically straight chain and contain at least 12, preferably 12 to 26, more preferably 18 to 22 carbon atoms. The most preferred unsaturated carboxylic acids are the C18 mono and/or diunsaturated carboxylic acids. The short chain saturated carboxylic acids are typically unbranched and contain 2 to 12, preferably 6 to 12, and most preferably 8 to 12 carbon atoms. The long chain saturated carboxylic acids are typically straight chain and contain at least 20, preferably 20 to 26, and most preferably 22 carbon atoms. The molar ratio of Group (a) carboxylic acid moieties to Group (b) carboxylic acid moieties in the solid polyol ester molecule is from 1:15 to 2:1, preferably 1:7 to 5:3, and more preferably 1:7 to 3:5. The average degree of esterification of these solid polyol carboxylic acid esters is such that at least 2 of the hydroxyl groups of the polyol are esterified. In the case of sucrose polyesters from 7 to 8 of the hydroxyl groups of the polyol are preferably esterified. Typically, substantially all, e.g., at least 85%, preferably at least 95%, of the hydroxyl groups of the polyol are esterified.

The polyols which are used in the solid polyol carboxylic acid ester compounds of the present invention preferably contain from 4 to 11, more preferably from 4 to 8, and most preferably from 6 to 8 hydroxyl groups.

Examples of preferred polyols of the solid polyol carboxylic acid esters are sugars, including monosaccharides and disaccharides and trisaccharides, and sugar alcohols containing from 4 to 11 hydroxyl groups. The trisaccharides raffinose and maltotriose are examples of sugars which contain 11 hydroxyl groups. The preferred sugars and sugar alcohols are those which contain 4 to 8, more preferably 6 to 8 hydroxyl groups. Examples of those containing four hydroxyl groups are the monosaccharides xylose and arabinose and the sugar alcohol erythritol. Suitable five hydroxyl group-containing polyols are the monosaccharides galatose, fructose, mannose and glucose, and the sugar alcohol xylitol. A polyol containing six hydroxyl groups is sorbitol. Examples of disaccharide polyols which can be used include maltose, lactose, and sucrose, all of which contain eight hydroxyl groups.

Examples of other suitable polyols are pentaerythritol, diglycerol, triglycerol, alkyl glycosides, and polyvinyl alcohols. The preferred polyol is sucrose.

Examples of long chain unsaturated carboxylic acid moieties for the solid polyol carboxylic acid esters herein are lauroleate, myristoleate, palmitoleate, oleate, elaidate, erucate, linoleate, linolenate, arachidonate, eicosapentaentoate, and docosahexaenoate. For oxidative stability, the mono- and disunsaturated fatty acid moieties are preferred.

Examples of suitable short chain saturated carboxylic acid moieties are acetate, caproate, caprylate, caprate and laurate.

Examples of suitable long chain saturated carboxylic acid moieties are arachidate, behenate, lignocerate, and cerotate.

Of course, the long chain unsaturated carboxylic acid moieties can be used singly or in mixtures with each other or in mixtures with the short chain saturated carboxylic acid moieties, in all proportions. Likewise, the long chain saturated carboxylic acid moieties can be used in combination with each other in all proportions. Mixed carboxylic acid moieties from source oils which contain substantial amounts of the desired unsaturated or saturated acids can be used as the acid moieties to prepare compounds of the present invention. The mixed carboxylic acids from the oils should contain at least 30%, preferably at least 50%, and most preferably at least 80% of the desired unsaturated or saturated acids. For example, rapeseed oil fatty acids or soybean oil fatty acids can be used instead of pure C12-C16 unsaturated fatty acids. Hardened, i.e. hydrogenated, high erucic rapeseed oil fatty acids can be used instead of pure C20-C26 saturated acids. Preferably the C20 and higher acids, or their derivatives, e.g. methyl of other low alkyl esters, are concentrated for example by distillation. The fatty acids from palm kernel oil or coconut oil can be used as a source of C8 to C12 acids. An example of the use of source oils to make solid polyol polyesters of the invention is the preparation of solid sucrose polyester, employing the fatty acids of high oleic sunflower oil and substantially completely hydrogenated high erucic rapeseed oil. When sucrose is substantially completely esterified with a 1:3 by weight blend of the methyl esters of the fatty acids of these two oils, the resulting sucrose polyester will have a molar ratio of unsaturated C18 acid radicals to C20 and higher saturated acid radicals of 1:1 and 28.6 weight percent of the total fatty acids in the polyester will be C22 fatty acids.

The higher the proportions of the desired unsaturated and saturated acids in the carboxylic acid stocks used in making the solid polyol polyester, the more efficient the ester will be in its ability to thicken or bind the liquid polyol esters.

The preferred unsaturated carboxylic acid moieties are those which have 18 carbon atoms, and are mono- and/or di-unsaturated. Preferred short chain carboxylic acid moieties are those which have 8-12 carbon atoms. The preferred long chain saturated carboxylic acid moiety is behenate. The preferred ratio of Group (a) fatty acid moieties to Group (b) fatty acid moieties is from 1:7 to 5:3, more preferably from 1:7 to 3:5. Preferred solid polyol polyesters of the invention are polyesters of sucrose in which at least 7 of the 8 hydroxyl groups are esterified.

Examples of solid polyol carboxylic acid polyesters of the present invention are sorbitol hexaester in which the carboxylic acid ester moieties are palmitoleate and arachidate in a 1:2 molar ratio: the octaester of raffinose in which the carboxylic acid ester moieties are linoleate and behenate in a 1:3 molar ratio: the heptaester of maltose wherein the esterifying carboxylic acid moieties are sunflower seed oil fatty acids and lignocerate in a 3:4 molar ratio: the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3:4 molar ratio. A preferred material is sucrose polyester in which the degree of esterification is 7-8, and in which the fatty acid moieties are C18 mono- and/or di-unsaturated and behenic, in a molar ratio of unsaturates:behenic of 1:7 to 3:5. A particularly preferred polyol ester thickening agent is the octaester of sucrose in which there are 7 behenic fatty acid moieties and 1 oleic acid moiety in the molecule.

The solid polyol carboxylic acid esters of the present invention can be made according to prior known methods for preparing polyesters of polyols. See, for example U.S. Patent No. 5,306,516, to Letton et al., issued April 26, 1994; U.S. Patent No. 5,306,515, to Letton et al., issued April 26, 1994; U.S. Patent No. 5,305,514, to Letton et al., issued April 26, 1994; U.S. Patent No. 4,797,300, to Jandacek et al., issued January 10, 1989; U.S. Patent No. 3,963,699, to Rizzi et al, issued June 15, 1976: U.S. Patent No. 4,518,772, to Volpenhein, issued May 21, 1985; and U.S. Patent No. 4,517,360, to Volpenhein, issued May 21, 1985.

### TOPICAL CARRIER

The present invention comprises from 0.1% to 99.9%, preferably from 50% to 99%, and more preferably from 60% to 95% by weight of a topical carrier for the skin conditioning agent and for any other optional components of the present invention.

The skin conditioning agents of the present invention can be formulated into a wide variety of product types, including creams, lotions, milks mousses, gels, lotions, tonics, sprays, hand and body lotions, cold creams, cleansing lotions, facial moisturizers, sunscreens, anti-acne preparations, topical analgesics, mascaras, lipsticks, and the like. The carriers and any additional components required to formulate such products vary with product type and can be routinely chosen by one skilled in the art.

The topical carrier can be in a wide variety of forms. For example, emulsion carriers, including, but not limited to, oil-in-water, water-in-oil, water-in-oil-in-water, and oil-in-water-in-silicone emulsions, are useful herein. These emulsions can cover a broad range of viscosities, e.g., from 100 cps to 200,000 cps. These emulsions can also be delivered in the form of sprays using either mechanical pump containers or pressurized aerosol containers using conventional propellants. These carriers can also be delivered in the form of a mousse. Other suitable topical carriers include anhydrous liquid solvents such as oils, alcohols, and silicones (e.g., mineral oil, ethanol, isopropanol, dimethicone, cyclomethicone, and the like); aqueous-based single phase liquid solvents (e.g., hydro-alcoholic solvent systems); and thickened versions of these anhydrous and aqueous-based single phase solvents (e.g., where the viscosity of the solvent has been increased to form a solid or semi-solid by the addition of appropriate gums, resins, waxes, polymers, salts, and the like). Examples of topical carrier systems useful in the present invention are described in the following references:

"Sun Products Formulary" Cosmetics & Toiletries, vol. 105, pp. 122-139 (December 1990); "Sun Products Formulary", Cosmetics & Toiletries, vol. 102, pp. 117-136 (March 1987); U.S. Patent No. 4,960,764 to Figueroa et al., issued October 2, 1990; U.S. Patent No. 4,254,105 to Fukuda et al., issued March 3, 1981; U.S. Patent No. 4,976,953, to Orr et al., issued December 11, 1990; and U.S. Patent No. 5,073,372, to Turner et al., issued December 17, 1991.

When the topical skin conditioning agent is an aerosol spray or mousse, the carrier can also utilize any of the conventional propellants to deliver the material as a foam (in the case of a mousse) or as a fine, uniform spray (in the case of an aerosol). Examples of suitable propellants include materials such as trichlorofluoromethane, dichlorodifluoromethane, difluoroethane, dimethylether, propane, n-butane or isobutane. A more complete disclosure of propellants useful herein can be found in Sagarin, Cosmetics Science and Technology, 2nd Edition, Vol. 2, pp. 443-465 (1972).

Suitable spray containers are well known in the art and include conventional, non-aerosol pump sprays i.e., "atomizers," aerosol containers or cans having propellant, as described above, and also pump aerosol containers utilizing compressed air as the propellent. Pump aerosol containers are disclosed, for example, in U.S. Patents 4,077,441, March 7, 1978, Olofsson and 4,850,577, July 25, 1989, and also in U.S. Serial No. 07/839,648, Gosselin, Lund, Sojka, and Lefebvre, filed February 21, 1992, "Consumer Product Package Incorporating A Spray Device Utilizing Large Diameter Bubbles. Pump aerosols hair sprays using compressed air are also currently marketed by The Procter & Gamble Company under their tradename VIDAL SASSOON AIRSPRAY hair sprays.

### Additional Components

A wide variety of additional components can be employed in the topical skin conditioning compositions herein. Non-limiting examples include the following:

### Pharmaceutical Actives

The compositions of the present invention can comprise a safe and effective amount of a pharmaceutical active. The phrase "safe and effective amount", as used herein, means an amount of an active high enough to significantly or positively modify the condition to be treated, but low enough to avoid serious side effects (at a reasonable benefit/risk ratio), within the scope of sound medical judgement. A safe and effective amount of the pharmaceutical active will vary with the specific active, the ability of the composition to penetrate the active through the skin, the amount of composition to be applied, the particular condition being treated, the age and physical condition of the patient being treated, the severity of the condition, the duration of the treatment, the nature of concurrent therapy, and like factors.

The pharmaceutical actives which can be used in the compositions of the present invention preferably comprise from 0.1% to 20% by weight of the compositions, more preferably from 0.1% to 10%, and most preferably from 0.1% to 5%. Mixtures of pharmaceutical actives may also be used.

Nonlimiting examples of pharmaceutical actives can include the following:

Useful pharmaceutical actives in the compositions of the present invention include anti-acne drugs. Anti-acne drugs for use in the present invention include the keratolytics such as salicylic acid sulfur, lactic acid, glycolic, pyruvic acid, resorcinol, and N-acetylcysteine; retinoids such as retinoic acid and its derivatives (e.g.. cis and trans); antibiotics and antimicrobials such as benzoyl peroxide, octopirox, erythromycin, zinc, tetracyclin, triclosan, azelaic acid and its derivatives, phenoxy ethanol and phenoxy propanol, ethylacetate, clindamycin and meclocycline; sebostats such as flavinoids; alpha and beta hydroxy acids; and bile salts such as scymnol sulfate and its derivatives, deoxycholate, and cholate. Preferred anti-acne actives are those selected from saliyclic acid, sulfur, resorcinol, lactic acid zinc, erythromycin, benzoyl peroxide, and mixtures thereof. More preferred for use is salicylic acid.

Useful pharmaceutical actives in the compositions of the present invention include non-steroidal anti-inflammatory drugs (NSAIDS). The NSAIDS can be selected from the following categories: propionic acid derivatives; acetic acid derivatives; fenamic acid derivatives; biphenylcarboxylic acid derivatives; and oxicams. All of these NSAIDS are fully described in the U.S. Patent 4,985,459 to Sunshine et al., issued January 15, 1991. Most preferred are the propionic NSAIDS including but not limited to aspirin, acetaminophen, ibuprofen, naproxen, benoxaprofen, flurbiprofen, fenoprofen, fenbufen, ketoprofer, indoprofen, pirprofen, carprofen, oxaprozin, pranoprofen, miroprofen, tioxaprofen, suprofen, alminoprofen, tiaprofenic acid, fluprofen and bucloxic acid. Also useful are the steroidal anti-inflammatory drugs including hydrocortisone and the like.

Useful pharmaceutical actives in the compositions of the present invention include antipruritic drugs. Antipruritic drugs preferred for inclusion in compositions of the present invention include pharmaceutically-acceptable salts of methdilizine and trimeprazine.

Useful pharmaceutical actives in the compositions of the present invention include include anesthetic drugs. Anesthetic drugs preferred for inclusion in compositions of the present invention include pharmaceutically-acceptable salts of lidocaine, bupivacaine, chlorprocaine, dibucaine, etidocaine, mepivacaine, tetracaine, dyclonine, hexylcaine, procaine, cocaine, ketamine, pramoxine and phenol.

Useful pharmaceutical actives in the compositions of the present invention include antimicrobial drugs (antibacterial, antifungal, antiprotozoal and antiviral drugs). Antimicrobial drugs preferred for inclusion in compositions of the present invention include pharmaceutically-acceptable salts of b-lactam drugs, quinolone drugs, ciprofloxacin, norfloxacin, tetracycline, erythromycin, amikacin, triclosan, doxycycline, capreomycin, chlorhexidine, chlortetracycline, oxyetracycline, clindamycin, ethambutol, metronidazole, pentamidine, gentamicin kanamycin. lineomycin, methacycline, methenamine, minocycline, neomycin, netilmicin, paromomycin, streptomycin, tobramycin miconazole and amanfadine. Antimicrobial drugs preferred for inclusion in compositions of the present invention include tetracycline hydrochloride, erythromycin estolate, erythromycin stearate (salt), amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethanbutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, amanfadine hydrochloride, amanfadine sulfate, triclosan, octopirox, parachlorometa xylenol, nystatin, tolnaftate and clotrimazole.

Also useful herein are sunscreening agents. A wide variety of sunscreening agents are described in U.S. Patent No. 5,087,445, to Haffey et al., issued February 11, 1992; U.S. Patent No. 5,073,372, to Turner et al., issued December 17, 1991; U.S. Patent No. 5,073,371, to Turner et al. issued December 17, 1991: and Segarin, et al., at Chapter VIII, pages 189 et seq., of Cosmetics Science and Technology. Preferred among those sunscreens which are useful in the compositions of the instant invention are those selected from 2-ethylhexyl p-methoxycinnitnate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide, and mixtures thereof.

Still other useful sunscreens are those disclosed in U.S. Patent No. 4,937,370, to Sabatelli, issued June 26, 1990; and U.S. Patent No. 4,999,186, to Sabatelli et al., issued March 12, 1991. The sunscreening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range. These sunscreening agents provide higher efficacy, broader UV absorption, lower skin penetration and longer lasting efficacy relative to conventional sunscreens. Especially preferred examples of these sunscreens include those selected from 4-N,N-(2-ethylhexyl)methylaminobenzoic acid ester of 2,4-dihydroxybenzophenone, 4-N,N-(2-ethylhexyl)methylaminobenzoic acid ester with 4-hydroxydibenzoylmethane, 4-N,N- (2-ethylhexyl)methylaminobenzoic acid ester of 2-hydroxy-4-(2-hydroxyethoxy)benzophenone, 4-N,N-(2-ethylhexyl)-methylaminobenzoic acid ester of 4-(2-hydroxyethoxy)dibenzoylmethane, and mixtures thereof.

Generally, the sunscreens can comprise from 0.5% to 20% of the compositions useful herein. Exact amounts will vary depending upon the sunscreen chosen and the desired Sun Protection Factor (SPF). SPF is a commonly used measure of photoprotection of a sunscreen against erythema. See Federal Register, Vol. 43, No. 166, pp. 38206-38269, August 25, 1978.

Also useful in the present invention are sunless tanning agents including dihydroxyacetone, glyceraldehyde, indoles and their derivatives, and the like. These sunless tanning agents can also be used in combination with the sunscreen agents.

Other useful actives include skin bleaching (or lightening) agents including but not limited to hydroquinone, ascorbic acid, kojic acid and sodium metabisulfite.

### Conventional Humectants and Moisturizers

The compositions of the present invention can also contain one or more conventional humectant or moisturizing materials. A variety of these materials can be employed and each can be present at a level from 0.1% to 20%, more preferably from 1% to 10% and most preferably from 2% to 5%. These materials include guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g.. aloe vera gel); polyhydroxy alcohols such as sorbitol, glycerol, hexanetriol, propylene glycol, butylene glycol, hexylene glycol and the like; polyethylene glycols; sugars and starches; sugar and starch derivatives (e.g., alkoxylated glucose); hyaluronic acid; lactamide monoethanolamine; acetamide monoethanolamine; and mixtures thereof.

### Emulsifiers

The compositions herein can contain various emulsifiers. These emulsifiers are useful for emulsifying the various carrier components of the compositions herein. Suitable emulsifiers can include any of a wide variety of nonionic cationic, anionic, and zwitterionic emulsifiers disclosed in the prior patents and other references. See McCutcheon's, Detergents and Emulsifiers, North American Edition (1986), published by Allured Publishing Corporation; U.S. Patent No. 5,011,681 to Ciotti et al., issued April 30, 1991; U.S. Patent No. 4,421,769 to Dixon et al., issued December 20, 1983; and U.S. Patent No. 3,755,560 to Dickert et al.. issued August 28, 1973.

Suitable emulsifier types include esters of glycerin, esters of propylene glycol, fatty acid esters of polyethylene glycol, fatty acid esters of polypropylene glycol, esters of sorbitol, esters of sorbitan anhydrides, carboxylic acid copolymers, esters and ethers of glucose, ethoxylated ethers. ethoxylated alcohols, alkyl phosphates polyoxyethylene fatty ether phosphates, fatty acid amides. acyl lactylates, soaps and mixtures thereof.

Suitable emulsifiers can include, but are not limited to, polyethylene glycol 20 sorbitan monolaurate (Polysorbate 20), polyethylene glycol 5 soya sterol, Steareth-20, Ceteareth-20, PPG-2 methyl glucose ether distearate. Ceteth-10, Polysorbate 80, cetyl phosphate, potassium cetyl phosphate, diethanolamine cetyl phosphate. Polysorbate 60, glyceryl stearate, PEG-100 stearate, and mixtures thereof.

The emulsifiers can be used individually or as a mixture of two or more and can comprise from 0.1% to 10%, more preferably from 1% to 7%, and most preferably from 1% to 5% of the compositions of the present invention.

### Carboxylic Acid Copolymer Thickeners

Another component useful in the compositions herein is a carboxylic acid copolymer thickener. These crosslinked polymers contain one or more monomers derived from acrylic acid, substituted acrylic acids, and salts and esters of these acrylic acids and the substituted acrylic acids, wherein the crosslinking agent contains two or more carbon-carbon double bonds and is derived from a polyhydric alcohol. The preferred polymers for use herein are of two general types. The first type of polymer is a crosslinked homopolymer of an acrylic acid monomer or derivative thereof (e.g., wherein the acrylic acid has substituents on the two and three carbon positions independently selected from the group consisting of C₁₋₄ alkyl, -CN, -COOH, and mixtures thereof). The second type of polymer is a crosslinked copolymer having a first monomer selected from an acrylic acid monomer or derivative thereof (as just described in the previous sentence), a short chain alcohol (i.e. a C₁₋₄) acrylate ester monomer or derivative thereof (e.g., wherein the acrylic acid portion of the ester has substituents on the two and three carbon positions independently selected from the group consisting of C₁₋₄ alkyl, -CN, -COOH, and mixtures thereof), and mixtures thereof and a second monomer which is a long chain alcohol (i.e. C₈₋₄₀) acrylate ester monomer or derivative thereof (e.g., wherein the acrylic acid portion of the ester has substituents on the two and three carbon positions independently selected from the group consisting of C₁₋₄ alkyl, -CN, -COOH, and mixtures thereof). Combinations of these two types of polymers are also useful herein.

In the first type of crosslinked homopolymers the monomers are preferably selected from acrylic acid, methacrylic acid, ethacrylic acid, and mixtures thereof, with acrylic acid being most preferred. In the second type of crosslinked copolymers the acrylic acid monomer or derivative thereof is preferably selected from acrylic acid, methacrylic acid, ethacrylic acid, and mixtures thereof, with acrylic acid, methacrylic acid, and mixtures thereof being most preferred. The short chain alcohol acrylate ester monomer or derivative thereof is preferably selected from C₁₋₄ alcohol acrylate esters, C₁₋₄ alcohol methacrylate esters. C₁₋₄ alcohol ethacrylate esters, and mixtures thereof, with the C₁₋₄ alcohol acrylate esters, C₁₋₄ alcohol methacrylate esters and mixtures thereof, being most preferred. The long chain alcohol acrylate ester monomer is selected from C₈₋₄₀ alkyl acrylate esters, with C₁₀₋₃₀ alkyl acrylate esters being preferred.

The crosslinking agent in both of these types of polymers is a polyalkenyl polyether of a polyhydric alcohol containing more than one alkenyl ether group per molecule, wherein the parent polyhydric alcohol contains at least 3 carbon atoms and at least 3 hydroxyl groups. Preferred crosslinkers are those selected from allyl ethers of sucrose and allyl ethers of pentaerythritol, and mixtures thereof. These polymers useful in the present invention are more fully described in U.S. Patent No. 5,087,445, to Haffey et al., issued February 11, 1992; U.S. Patent No. 4,509,949, to Huang et al., issued April 5, 1985; U.S. Patent No. 2,798,053, to Brown, issued July 2, 1957. See also, CTFA International Cosmetic Ingredient Dictionary, fourth edition, 1991, pp. 12 and 80.

Examples of commercially available homopolymers of the first type useful herein include the carbomers, which are homopolymers of acrylic acid crosslinked with allyl ethers of sucrose or pentaerytritol. The carbomers are available as the Carbopol® 900 series from B.F. Goodrich. Examples of commercially available copolymers of the second type useful herein include copolymers of C₁₀₋₃₀ alkyl acrylates with one or more monomers of acrylic acid, methacrylic acid or one of their short chain (i.e. C₁₋₄ alcohol) esters, wherein the crosslinking agent is an allyl ether of sucrose or pentaerytritol. These copolymers are known as acrylates/C10-30 alkyl acrylate crosspolymers and are commercially available as Carbopol® 1342, Pemulen TR-1, and Pemulen TR-2, from B.F. Goodrich. In other words, examples of carboxylic acid polymer thickeners useful herein are those selected from carbomers, acrylates/C10-C30 alkyl acrylate crosspolymers, and mixtures thereof.

The compositions of the present invention can comprise from 0.025% to 1%, more preferably from 0.05% to 0.75% and most preferably from 0.10% to 0.50% of the carboxylic acid polymer thickeners.

### Oils

The compositions of the present invention can also optionally comprise various oil materials, that is, a material generally having low solubility in water, generally less than 1% by weight at 25°C. Examples of suitable oil components include, but are not limited to, volatile and non-volatile silicone oils, highly branched hydrocarbons, and non-polar carboxylic acid and alcohol esters, and mixtures thereof. Oils useful in the instant invention are further described in U.S. Patent No. 4,919,934, to Deckner et al., issued April 24 1990.

Volatile silicone components such as cyclic polydimethylsiloxanes containing from 3 to 9 silicon atoms, and dimethicone are useful herein. Nonvolatile silicones include polyalkylsiloxanes and polyalkylaryl siloxanes. Useful volatile and nonvolatile silicones are disclosed in U.S. Patent No. 5,069,897, to Orr, issued December 3, 1991.

### Other Additional Components

The compositions of the present invention can comprise a wide range of other additional components. The CTFA Cosmetic Ingredient Handbook, Second Edition, 1992, describes a wide variety of nonlimiting cosmetic and pharmaceutical ingredients commonly used in the skin care industry, which are suitable for use in the compositions of the present invention. Nonlimiting examples of functional classes of ingredients are described at page 537 of this reference. Examples of these functional classes include: absorbents, abrasives, anti-acne agents, anticaking agents, antifoaming agents, antimicrobial agents, antioxidants, binders, biological additives, buffering agents, bulking agents, chelating agents, chemical additives, colorants, cosmetic asringents, cosmetic biocides, denaturants, drug astringents, external analgesics, film formers, fragrance components, humectants, opacitying agents, pH adjusters, plasticizers, preservatives, propellants, reducing agents, skin protectants, solvents, suspending agents (nonsurfactant), ultraviolet light absorbers, and viscosity increasing agents (aqueous and nonaqueous). Examples of other functional classes of materials useful herein that are well known to one of ordinary skill in the art include emulsifiers, solubilizing agents, and sequestrants, and the like.

Nonlimiting examples of these additional components cited in the CTFA Cosmetic Ingredient Handbook, as well as other materials useful herein, include the following: vitamins and derivatives thereof [e.g., vitamin C, Vitamin A (i.e. retinoic acid), retinol, retinoids, and the like]; anti-oxidants; polyethyleneglycols and polypropyleneglycols; polymers for aiding the film-forming properties and substantivity of the composition (such as a copolymer of eicosene and vinyl pyrrolidone, in example of which is available from GAF Chemical Corporation as Ganex® V-220); preservatives for maintaining the antimicrobial integrity of the compositions; antioxidants; chelators and sequestrants; crosslinked and noncrosslinked nonionic and cationic polyacrylamides [e.g., Salcare SC92 which has the CTFA designation polyquaternium 32 (and) mineral oil, and Salcare SC 95 which has the CTFA designation polyquaternium 37 (and) mineral oil (and) PPG-1 trideceth6, and the nonionic Seppi-Gel polyacrylamides available from Seppic Corp.]; and aesthetic components such as fragrances, pigments, colorings, essential oils, skin senates, astringents, skin soothing agents, skin healing agents and the like, nonlimiting examples of these aesthetic components include clove oil, menthol, camphor, eucalyptus oil, eugenol, menthyl lactate, witch hazel distillate, bisabolol, dipotassium glycyrrhizinate and the like.

### METHODS OF CONDITIONING THE SKIN

The skin conditioning compositions of the present invention are used in conventional ways to provide a skin conditioning benefit to the skin, and to provide any additional cosmetic or pharmaceutical benefits appropriate to the product such as sun protection, anti-acne benefits, anti-wrinkle and anti-skin aging benefits, artificial tanning, analgesic benefits, and the like. Such methods of use depend upon the type of composition employed but generally involve application of an effective amount of the product to the skin. By "effective amount" is meant an amount sufficient to provide the benefit desired. Typical amounts of the compositions of the present invention which are applied to the skin will vary depending upon the type of composition and the benefit desired, however, typical ranges are generally from 0.1 mg/cm² to 25 mg/cm², with 2 mg/cm² being typical.

### EXAMPLES

The following examples further describe and demonstrate embodiments within the scope of the present invention.

Ingredients are identified by chemical or CTFA name.

### EXAMPLE 1

### Moisturizer

A moisturizer is prepared by combining the following ingredients using conventional mixing techniques.

| Ingredients | Weight Percent |
|---|---|
| Water | qs100 |
| Cetyl Alcohol | 1.80 |
| Stearic Acid | 0.25 |
| Stearyl Alcohol | 1.20 |
| PEG 100-stearate | 0.25 |
| Isopropyl Palmitate | 1.00 |
| Cetyl Ricinoleate | 1.00 |
| Thickened Sucrose Polyester¹ | 4.50 |
| Dimethicone 350² | 0.50 |
| Propyl Paraben | 0.10 |
| Arlatone (RTM) 2121³ | 1.00 |
| Glycerin | 9.00 |
| Urea | 2.00 |
| Octyl Methoxycinnamate | 2.00 |
| Phenoxyethanol | 0.25 |
| Carbomer 1382⁴ | 0.05 |
| Carbomer 954⁵ | 0.35 |
| Tetrasodium EDTA | 0.10 |
| Titanium Dioxide | 0.15 |
| Methyl Paraben | 0.20 |
| NaOH | 0.22 |
| Dimethicone Q-21403⁶ | 1.00 |

| | |
|---|---|
| ¹ The thickened sucrose polyester is a mixture of approximately 82.3% by weight of mixture of mixed hexa-, hepta-, and octa-sucrose esters, predominately the octa-ester esterified with mixed soybean oil fatty acids, and 17.7% by weight of a sucrose octaester esterified with 1 oleic acid and 7 behenic acid moieties. | |
| ² Dow Corning® 200 Fluid (350 centistoke) from Dow Corning. | |
| ³ 95% by weight sorbitan stearate and 5% by weight sucrose cocoate. | |
| ⁴ Carbopol® 1382 from B.F. Goodrich. | |
| ⁵ Carbopol® 954 from B.F. Goodrich. | |
| ⁶ Dow Corning® Q-2 1403 from Dow Corning which is a mixture of 85% by weight dimethicone and 15% by weight dimethiconal. | |

The composition is made as follows:

A first premix of thickening agents, Arlatone 2121 and other water soluble ingredients is prepared by admixing in water and heating. A second premix of oil phase ingredients other than the silicones is prepared by mixing and heating and is added to the aqueous premix.

The resulting mixture is cooled. The silicones are then added to the resulting oil-in-water emulsion and the mixture is cooled before adding minor ingredients. The composition is ready for packaging.

The composition is useful for topical application to skin and displays improved moisturization, skin feel and skin care characteristics together with reduced greasiness and excellent rub-in absorption characteristics.

### EXAMPLE 2

### Moisturizer

A moisturizer is prepared by combining the following ingredients using conventional mixing techniques.

| | Weight % |
|---|---|
| Ingredients | Example 2 |
| Water | qs100 |
| Cetyl Alcohol | 1.80 |
| Stearic Acid | 0.25 |
| Stearyl Alcohol | 1.20 |
| Peg 100-stearate | 0.25 |
| Mineral Oil | 2.00 |
| Isopropyl Palmitate | 1.00 |
| Thickened Sucrose Polyester¹ | 4.50 |
| Dimethicone 350² | 0.50 |
| Propyl Paraben | 0.10 |
| Arlatone (RTM) 2121³ | 1.00 |
| Glycerin | 9.00 |
| Urea | 2.00 |
| Octyl Methoxycinnamate | 2.00 |
| Phenoxyethanol | 0.25 |
| Carbomer 1382⁴ | 0.05 |
| Carbomer 954⁵ | 0.35 |
| Tetrasodium EDTA | 0.10 |
| Titanium Dioxide | 0.15 |
| Methyl Paraben | 0.20 |
| NaOH | 0.22 |
| Dimethicone Q-21403⁶ | 1.00 |

| | |
|---|---|
| ¹ The thickened sucrose polyester is a mixture of approximately 82.3% by weight of mixture of mixed hexa-, hepta-, and octa-sucrose esters, predominately the octa-ester esterified with mixed soybean oil fatty acids, and 17.7% by weight of a sucrose octaester esterified with 1 oleic acid and 7 behenic acid moieties. | |
| ² Dow Corning® 200 Fluid (350 centistoke) from Dow Corning. | |
| ³ 95% by weight sorbitan stearate and 5% by weight sucrose cocoate. | |
| ⁴ Carbopol® 1382 from B.F. Goodrich. | |
| ⁵ Carbopol® 954 from B.F. Goodrich. | |
| ⁶ Dow Corning® Q-2 1403 from Dow Corning, which is a mixture of 85% by weight dimethicone and 15% by weight dimethiconal. | |

The composition is made as follows:

A first premix of thickening agents, Arlatone 2121 and other water soluble ingredients is prepared by admixing in water and heating. A second premix of oil phase ingredients other than the silicones is prepared by mixing and heating and is added to the aqueous premix.

The resulting mixture is cooled. The silicones are then added to the resulting oil-in-water emulsion and the mixture is cooled before adding minor ingredients. The composition is ready for packaging.

These compositions are useful for topical application to skin and displays improved moisturization, skin feel and skin care characteristics together with reduced greasiness and excellent rub-in absorption characteristics.

### EXAMPLE 3

### Sunscreen

A sunscreen is prepared by combining the following ingredients using conventional mixing techniques.

| Ingredients | Weight Percent |
|---|---|
| Water | qs100 |
| Octyl Methoxycinnamate | 7.50 |
| Octocrylene | 3.75 |
| Oxybenzone | 2.00 |
| 1,3, Dihydroxyacetone | 3.00 |
| Thickened Sucrose Polyester¹ | 2.00 |
| Butylene Glycol | 2.00 |
| Salcare SC95² | 1.25 |
| Ganex V-220³ | 1.00 |
| Permethyl 101a⁴ | 1.00 |
| Fragrance | 0.50 |
| Cetyl Palmitate | 0.75 |
| Synchrowax HRC⁵ | 0.75 |
| Cetyl Alcohol | 0.50 |
| Glydant Plus | 0.20 |
| Varisoft TA-100⁶ | 0.20 |
| Natrosol Plus CS 330⁷ | 0.20 |
| Disodium EDTA | 0.05 |

| | |
|---|---|
| ¹ The thickened sucrose polyester is a mixture of approximately 82.3% by weight of mixture of mixed hexa-, hepta-, and octa-sucrose esters, predominately the octa-ester esterified with mixed soybean oil fatty acids, and 17.7% by weight of a sucrose octaester esterified with 1 oleic acid and 7 behenic acid moieties. | |
| ² Polyquaternium-37, mineral oil, and PPG-1 trideceth-6, available from Allied Colloids, Norfolk. VA. | |
| ³ PVP/Eicosene copolymer. | |
| ⁴ Isohexadecane. | |
| ⁵ Tribehenin. | |
| ⁶ Distearyldimonium chloride. | |
| ⁷ Cetyl hydroxyethylcellulose. | |

The composition is made as follows:

A first premix of thickening agents and other water soluble ingredients is prepared by admixing in water and heating. A second premix of oil phase ingredients is prepared by mixing and heating and is added to the aqueous premix.

The resulting oil-in-water emulsion is cooled before adding minor ingredients. The composition is ready for packaging.

This composition is useful for topical application to the skin as a sunscreen composition and displays improved moisturization, skin feel and skin care characteristics together with reduced greasiness and excellent rub-in absorption characteristics.

### EXAMPLE 4

### Anti-Acne Gel

An anti-acne gel is prepared by combining the following ingredients using conventional mixing techniques.

| Ingredients | Weight Percent |
|---|---|
| Water | qs100 |
| Thickened Sucrose Polyester | 2.00 |
| Benzoyl Peroxide² | 2.50 |
| Carbomer 980³ | 0.30 |
| Glydant Plus⁴ | 0.20 |
| Acrylates/C10-30 Alkylacrylates crosspolymer⁵ | 0.05 |
| Disodium EDTA | 0.10 |
| Stearyl Alcohol | 2.25 |
| Cetyl Alcohol | 2.25 |
| Glycerylhydroxy Stearate | 0.74 |
| Steareth 100 | 0.50 |
| Sucrose Polyester | 2.50 |
| Sodium Hydroxide | 0.05 |
| Dimethicone⁶ | 0.60 |
| Cyclomethicone/dimethiconal⁷ | 0.50 |

| | |
|---|---|
| ¹ The thickened sucrose polyester is a mixture of approximately 82.3% by weight of mixture of mixed hexa-, hepta-, and octa-sucrose esters, predominately the octa-ester esterified with mixed soybean oil fatty acids, and 17.7% by weight of a sucrose octaester esterified with 1 oleic acid and 7 behenic acid moieties. | |
| 2 Lucidol® 75 from Elf Atochem, which is a powder containing 75% benzoyl peroxide active. | |
| 3 Carbopol® 980 from B.F. Goodrich. | |
| 4 DMDM Hydantoin (and) Iodopropynyl Butylcarbamate. | |
| 5 Pemulen® TR-1 from B.F. Goodrich. | |
| 6 Dow Corning® 200 Fluid (350 centistoke) from Dow Corning. | |
| 7 Dow Corning® Q-2 1401 from Dow Corning. | |

The composition is made as follows:

In a suitable vessel a benzoyl peroxide slurry is prepared by combining the benzoyl peroxide with water which accounts for approximately 3.6% of the batch. This slurry is passed through a Colloid or Urschel mill to disperse the benzoyl peroxide and the mill is rinsed through with an additional 1.44% of water. This rinse is added to the total slurry.

In a separate vessel a 5% sodium hydroxide solution is prepared with water to provide sodium hydroxide to the batch at .05%. In another vessel, the Carbomer 980 is gradually combined with an amount of water totaling 14.7% of the batch. It is added under agitation to disperse and hydrate the carbomer.

In a suitable mixing tank, the remaining water is added and heated to at least 75°C. In a separate vessel, the dimethicone, cetyl alcohol, stearyl alcohol, glycerylhydroxy stearate, thickened sucrose polyester, and Steareth 100 are added and heated to at least 75°C. As the water phase is heating, the disodium EDTA, Glydant Plus, and alkyl acrylates are added and mixed until dissolved.

When both phases reach the required temperature, the oil phase is slowly added to the water phase while the entire batch is recycled through a tekmar mill to reduce the oil droplet particle size to approximately one to two microns. The batch is then cooled to room temperature under constant agitation.

When the batch has cooled, the carbopol slurry, benzoyl peroxide slurry, and the cyclomethicone/dimethiconal are added. The batch is again recycled through the tekmar mill to disperse the materials. Finally, the 5% NaOH solution is gradually added with continuous mixing. The composition is then mixed until homogeneous.

This composition is useful for topical application to the skin as an anti-acne composition and displays improved moisturization, skin feel and skin care characteristics together with reduced greasiness and excellent rub-in absorption characteristics.

### EXAMPLE 5

### Lipstick

A moisturizing lipstick is prepared by combining the following ingredients using conventional mixing techniques.

| Ingredients | Weight Percent |
|---|---|
| Thickened Sucrose Polyester ¹ | 85 |
| Pigments² | QS100 |

| | |
|---|---|
| ¹ The thickened sucrose polyester is a mixture of approximately 82.3% by weight of mixture of mixed hexa-, hepta-, and octa-sucrose esters, predominately the octa-ester esterified with mixed soybean oil fatty acids, and 17.7% by weight of a sucrose octaester esterified with 1 oleic acid and 7 behenic acid moieties. | |
| ² A wide variety of standard pigments can be utilized depending upon the color desired. For example a mixture of titanium dioxide, red iron oxide, red 7 calcium lake, and yellow 5 aluminum lake can be used in equal weight proportions. | |

The composition is made as follows:

The thickened sucrose polyester is heated with mixing until a transparent liquid is formed. The pigments are then mixed in and the system is placed in the molds. The system is then cooled forming the lipstick.

This composition is useful for topical application to the lips as a lipstick and displays improved moisturization, skin feel and skin care characteristics together with reduced greasiness.

## Claims

1. A topical skin care composition comprising:
(A) from 0.1% to 99.9% of a skin conditioning agent comprising:
(i) from 50% to 99.99%, preferably from 80% to 98%, based on the total weight of said skin conditioning agent, of a nonocclusive, liquid polyol carboxylic acid ester having a polyol moiety and at least 2 carboxylic acid moieties, preferably 4 carboxylic acid moieties, wherein the polyol moiety is selected from sugars and sugar alcohols containing from 4 to 11 hydroxyl groups, and wherein each carboxylic acid moiety has from 8 to 22 carbon atoms, preferably from 14 to 18 carbon atoms, and wherein said liquid ester has a complete melting point of less than 30°C; and
(ii) from 0.01% to 50%, preferably from 2% to 20%, based on the total weight of said skin conditioning agent, of a solid polyol carboxylic acid ester having a polyol moiety and at least 2 carboxylic acid moieties wherein the polyol moiety contains at least 4 hydroxyl groups, wherein the carboxylic acid moiety consists essentially of (a) C12 or higher unsaturated carboxylic acid moieties or a mixture of said unsaturated moieties and C2 to C12 saturated carboxylic acid moieties, and (b) C20 or higher saturated carboxylic acid moieties, the molar ratio of (a) to (b) being from 1:7 to 3:5, wherein at least 2 of the hydroxyl groups of the polyol moiety are esterified with carboxylic acid moieties; and wherein the complete melting point of the solid polyol carboxylic acid ester is above 30°C; and
(B) from 0.1% to 99.9% of a topical carrier for said skin conditioning agent.

2. A composition according to Claim 1 wherein said polyol moiety of said liquid polyol carboxylic acid ester is selected from erythritol, xylitol, sorbitol, glucose, sucrose, and mixtures thereof, and is preferably sucrose.

3. A composition according to Claim 2 wherein said liquid carboxylic acid polyol ester is selected from sucrose pentaoleate, sucrose hexaoleate, sucrose heptaoleate, sucrose octaoleate, and mixtures thereof.

4. A composition according to Claim 3 wherein said polyol moiety of said solid polyol carboxylic acid ester has from 4 to 8 hydroxyl groups and said unsaturated carboxylic acid moieties of said solid polyol carboxylic acid ester have from 12 to 26 carbon atoms, and said saturated fatty acid moiety of said solid polyol carboxylic acid ester has from 20 to 26 carbon atoms.

5. A composition according to Claim 4 wherein said polyol moiety of said solid polyol carboxylic acid polyester is a sugar or sugar alcohol, preferably a sugar or sugar alcohol having from 6 to 8 hydroxyl groups, preferably sucrose.

6. A composition according to Claim 5 wherein said saturated carboxylic acid moieties of said solid polyol carboxylic acid ester consists essentially of behenic moieties.

7. A composition according to Claim 6 wherein said solid polyol carboxylic acid polyester is a sucrose octaester having one oleic acid moiety and seven behenic acid moieties.

8. A composition according to Claim 1 which further comprises a pharmaceutical active selected from anti-acne drugs, non-steroidal anti-inflammatory drugs, antipruritic drugs, anesthetic drugs, antimicrobial drugs, sunscreening agents, sunless tanning agents, skin bleaching agents, and mixtures thereof.

9. A composition according to Claim 8 wherein said active is an anti-acne active selected from salicylic acid, sulfur, lactic acid, zinc, erythromycin, benzoyl peroxide, and mixtures thereof.

10. A non-therapeutic method of conditioning skin in humans comprising topically applying to a human in need of treatment a safe and effective amount of a composition according to Claim 1.

## Patentansprüche

1. Topische Hautpflegezusammensetzung, umfassend:
(A) 0,1 % bis 99,9 % von einem hautkonditionierenden Mittel, umfassend:
(i) 50 % bis 99,99 %, vorzugsweise 80 % bis 98 %, bezogen auf das Gesamtgewicht des genannten hautkonditionierenden Mittels, von einem nichtokklusiven, flüssigen Polyolcarbonsäureester mit einem Polyolrest und mindestens 2 Carbonsäureresten, vorzugsweise 4 Carbonsäureresten, worin der Polyolrest unter Zuckern und Zuckeralkoholen mit 4 bis 11 Hydroxylgruppen ausgewählt ist, und worin jeder Carbonsäurerest 8 bis 22 Kohlenstoffatome, vorzugsweise 14 bis 18 Kohlenstoffatome aufweist, und worin der genannte flüssige Ester einen Schmelzpunkt des vollständigen Schmelzens von weniger als 30°C besitzt; und
(ii) 0,01 % bis 50 %, vorzugsweise 2 % bis 20 %, bezogen auf das Gesamtgewicht des genannten hautkonditionierenden Mittels, von einem festen Polyolcarbonsäureester mit einem Polyolrest und mindestens 2 Carbonsäureresten, worin der Polyolrest mindestens 4 Hydroxylgruppen aufweist, worin der Carbonsäurerest im wesentlichen aus (a) C₁₂- oder höheren ungesättigten Carbonsäureresten oder einem Gemisch aus den genannten ungesättigten Resten und gesättigten C₂-C₁₂-Carbonsäureresten und (b) C₂₀- oder höheren gesättigten Carbonsäureresten besteht, wobei das Molverhältnis von (a) zu (b) von 1:7 bis 3:5 beträgt, worin mindestens 2 der Hydroxylgruppen des Polyolrestes mit Carbonsäureresten verestert sind; und worin der Schmelzpunkt des vollständigen Schmelzens des festen Polyolcarbonsäureesters über 30°C liegt; und
(B) 0,1 % bis 99,9 % von einem topischen Träger für das genannte hautkonditionierende Mittel.

2. Zusammensetzung nach Anspruch 1, worin der genannte Polyolrest des genannten flüssigen Polyolcarbonsäureesters unter Erythrit, Xylit, Sorbit, Glucose, Saccharose und Gemischen hievon ausgewählt und vorzugsweise Saccharose ist.

3. Zusammensetzung nach Anspruch 2, worin der genannte flüssige Carbonsäurepolyolester unter Saccharosepentaoleat, Saccharosehexaoleat, Saccharoseheptaoleat, Saccharoseoctaoleat und Gemischen hievon ausgewählt ist.

4. Zusammensetzung nach Anspruch 3, worin der genannte Polyolrest des genannten festen Polyolcarbonsäureesters 4 bis 8 Hydroxylgruppen besitzt und die genannten ungesättigten Carbonsäurereste des genannten festen Polyolcarbonsäureesters 12 bis 26 Kohlenstoffatome aufweisen und der genannte gesättigte Fettsäurerest des genannten festen Polyolcarbonsäureesters 20 bis 26 Kohlenstoffatome besitzt.

5. Zusammensetzung nach Anspruch 4, worin der genannte Polyolrest des genannten festen Polyolcarbonsäurepolyesters ein Zucker oder ein Zuckeralkohol, vorzugsweise ein Zucker oder ein Zuckeralkohol mit 6 bis 8 Hydroxylgruppen, vorzugsweise Saccharose, ist.

6. Zusammensetzung nach Anspruch 5, worin die genannten gesättigten Carbonsäurereste des genannten festen Polyolcarbonsäureesters im wesentlichen aus Behensäureresten bestehen.

7. Zusammensetzung nach Anspruch 6, worin der genannte feste Polyolcarbonsäurepolyester ein Saccharoseoctaester mit einem Ölsäurerest und sieben Behensäureresten ist.

8. Zusammensetzung nach Anspruch 1, welche ferner ein pharmazeutisch wirksames Mittel umfaßt, welches unter Arzneimitteln gegen Akne, nichtsteroidalen entzündungshemmenden Arzneimitteln, Antipruriginosa, Anästhetika, antimikrobiellen Arzneimitteln, Sonnenschutzmitteln, Mitteln für ein sonnenloses Bräunen, hautbleichenden Mitteln und Gemischen hievon ausgewählt ist.

9. Zusammensetzung nach Anspruch 8, worin das genannte wirksame Mittel ein Mittel gegen Akne ist, welches unter Salicylsäure, Schwefel, Milchsäure, Zink, Erythromycin, Benzoylperoxid und Gemischen hievon ausgewählt ist.

10. Ein nicht-therapeutisches Verfahren zur Konditionierung menschlicher Haut, umfassend das topische Aufbringen einer sicheren und wirksamen Menge einer Zusammensetzung nach Anspruch 1 bei einem Menschen, welcher einer derartigen Behandlung bedarf.

## Revendications

1. Composition de soin pour la peau à usage local comprenant:
(A) de 0,1% à 99,9% d'un agent de conditionnement de la peau comprenant:
(i) de 50% à 99,99%, de préférence de 80% à 98%, par rapport au poids total dudit agent de conditionnement de la peau, d'un ester d'acide carboxylique et de polyol liquide, non occlusif, possédant un groupement polyol et au moins 2 groupements acides carboxyliques, de préférence 4 groupements acides carboxyliques, dans lequel le groupement polyol est choisi parmi les sucres et les alcools de sucres contenant de 4 à 11 groupes hydroxyle, et dans lequel chaque groupement acide carboxylique comporte de 8 à 22 atomes de carbone, de préférence de 14 à 18 atomes de carbone, ledit ester liquide possédant un point de fusion complète inférieur à 30°C; et
(ii) de 0,01% à 50%, de préférence de 2% à 20%, par rapport au poids total dudit agent de conditionnement de la peau, d'un ester d'acide carboxylique et de polyol solide possédant un groupement polyol et au moins 2 groupements acides carboxyliques, dans lequel le groupement polyol contient au moins 4 groupes hydroxyle, dans lequel le groupement acide carboxylique est constitué essentiellement (a) de groupements acides carboxyliques insaturés en C12 ou plus ou d'un mélange desdits groupements insaturés et de groupements acides carboxyliques saturés en C2 à C12, et (b) de groupements acides carboxyliques saturés en C20 ou plus, le rapport molaire de (a) à (b) étant de 1:7 à 3:5, où au moins 2 des groupes hydroxyle du groupement polyol sont estérifiés avec des groupements acides carboxyliques; le point de fusion complète de l'ester d'acide carboxylique et de polyol solide étant supérieur à 30°C; et
(B) de 0,1% à 99,9% d'un véhicule pour usage local, pour ledit agent de conditionnement de la peau.

2. Composition selon la revendication 1, dans laquelle ledit groupement polyol dudit ester d'acide carboxylique et de polyol liquide est choisi parmi l'érythritol, le xylitol, le sorbitol, le glucose, le saccharose, et leurs mélanges, et est de préférence, le saccharose.

3. Composition selon la revendication 2, dans laquelle ledit ester de polyol et d'acide carboxylique liquide est choisi parmi le pentaoléate de saccharose, l'hexaoléate de saccharose, l'heptaoléate de saccharose, l'octaoléate de saccharose, et leurs mélanges.

4. Composition selon la revendication 3, dans laquelle ledit groupement polyol dudit ester d'acide carboxylique et de polyol solide possède de 4 à 8 groupes hydroxyle et lesdits groupements acides carboxyliques insaturés dudit ester d'acide carboxylique et de polyol solide comportent de 12 à 26 atomes de carbone, et ledit groupement acide gras saturé dudit ester d'acide carboxylique et de polyol solide comporte de 20 à 26 atomes de carbone.

5. Composition selon la revendication 4, dans laquelle ledit groupement polyol dudit polyester d'acide carboxylique et de polyol solide est un sucre ou un alcool de sucre, de préférence un sucre ou un alcool de sucre possédant de 6 à 8 groupes hydroxyle, de préférence le saccharose.

6. Composition selon la revendication 5, dans laquelle lesdits groupements acides carboxyliques saturés dudit ester d'acide carboxylique et de polyol solide sont essentiellement constitués de groupements béhéniques.

7. Composition selon la revendication 6, dans laquelle ledit polyester d'acide carboxylique et de polyol solide est un octaester de saccharose possédant un groupement acide oléique et sept groupements acides béhéniques.

8. Composition selon la revendication 1, qui comprend en outre une substance pharmaceutique active choisie parmi les médicaments anti-acnéiques, les médicaments anti-inflammatoires non stéroïdiens, les médicaments antiprurigineux, les médicaments anesthésiques, les médicaments antimicrobiens, les agents écran solaire, les agents autobronzants, les agents de blanchment de la peau, et leurs mélanges.

9. Composition selon la revendication 8, dans laquelle ladite substance active est une substance active anti-acnéique choisie parmi l'acide salicylique, le soufre, l'acide lactique, le zinc, l'érythromycine, le peroxyde de benzoyle, et leurs mélanges.

10. Procédé non thérapeutique de conditionnement de la peau chez les humains, comprenant l'application locale à un humain nécessitant un traitement, d'une quantité sans danger et efficace d'une composition selon la revendication 1.
